(19) **Europäisches Patentamt**
**European Patent Office**
**Office européen des brevets**

(11) **EP 3 431 079 B1**

(12) **EUROPEAN PATENT SPECIFICATION**

(45) Date of publication and mention
of the grant of the patent:
**07.06.2023 Bulletin 2023/23**

(21) Application number: **17766652.6**

(22) Date of filing: **14.03.2017**

(51) International Patent Classification (IPC):
**A61K 9/20** *(2006.01)* **A61K 9/00** *(2006.01)*
**A61J 3/00** *(2006.01)*

(52) Cooperative Patent Classification (CPC):
**A61K 9/2072; A61K 9/2095**

(86) International application number:
**PCT/JP2017/010096**

(87) International publication number:
**WO 2017/159653 (21.09.2017 Gazette 2017/38)**

(54) **TABLET**

TABLETTE

COMPRIMÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2016 JP 2016050893**

(43) Date of publication of application:
**23.01.2019 Bulletin 2019/04**

(73) Proprietor: **Astellas Pharma Inc.**
**Chuo-ku**
**Tokyo 103-8411 (JP)**

(72) Inventors:
• **HATTORI, Shota**
**Tokyo 103-8411 (JP)**
• **IKEDA, Sorato**
**Tokyo 103-8411 (JP)**
• **KOBAYASHI, Masanori**
**Tokyo 103-8411 (JP)**

(74) Representative: **Gille Hrabal**
**Partnerschaftsgesellschaft mbB**
**Patentanwälte**
**Brucknerstraße 20**
**40593 Düsseldorf (DE)**

(56) References cited:
**WO-A1-99/05120          WO-A1-03/026627**
**JP-A- H03 123 726      JP-A- S57 501 847**
**JP-A- 2001 504 509     JP-A- 2004 196 674**
**JP-A- 2005 508 329     US-A- 2 386 416**
**US-A- 2 386 416**

• **Debra Catterson ET AL: "Guidance for Industry
Size, Shape, and Other Physical Attributes of
Generic Tablets and Capsules", , 1 December
2013 (2013-12-01), XP055574568, Retrieved from
the Internet:
URL:https://www.fdanews.com/ext/resources/
files/12/12-09-13-Guidance.pdf [retrieved on
2019-03-26]**

## EP 3 431 079 B1

**Description**

TECHNICAL FIELD

The present invention relates to a tablet, and more particularly to a tablet for oral administration.

BACKGROUND ART

In the related art, a tablet for oral administration has sizes of about 6 to 15 mm in diameter and about 100 to 500 mg in weight, and has a shape of a round type or an oval type is common, in consideration of easiness of pinching and easiness of swallowing.

However, there is a reported problem that, for an elderly person or a patient (for example, a rheumatoid arthritis patient) who has a limited range of joint motion including hand movement, even in the case of the tablet having the above size or shape, the tablet cannot be pinched and cannot be picked up when it has slipped out of hands (Owaki Naoko, A Multi-center Study on Convenience of Removing Tablets and Capsules from Heat-sealed Packages, medical medicine, 30(5) 312-320(2004)).

Further, in the case of round type tablets, there is also a problem in which the tablet easily rolls and is lost.

Amid such circumstances, various tablets have been proposed, which are hard to roll when dropped off by devising the shape of the tablets, and which facilitate pinching even for an elderly person (see, for example, Patent Literature 1 and 2).

The tablet described in Patent Literature 1 is an oval type tablet, and includes a tablet body portion having a shape biased in a predetermined direction, and a convex portion continuously protruding upward from an upper surface of the tablet body portion.

With the above configuration, when the elderly person takes out a tablet, even if the tablet falls on a table or on a floor, it is possible to prevent the tablet from rolling or missing.

Also, if the tablet is placed in a shape with the convex portion facing downward, the height of the tablet will be somewhat higher, making it easier to pinch the tablet somewhat.

In addition, a tablet described in Patent Literature 2 is a round type and has a shape in which a flat inclined surface is formed by cutting a part of the tablet body portion.

With the above-described configuration, when the tablet rolls, rotates and moves, the balance tends to collapse and the tablet is hard to roll. Also, since protruding portions (convex portions) protruding from the tablet body portion are not formed, easiness of swallowing is also ensured for the time being.

Also, if the tablet is placed in a shape with the inclined surface portion facing downward, the height of the tablet will be somewhat higher, making it easier to pinch the tablet somewhat. US2386416 discloses a tablet having two protruding portions which are protruding in opposite directions.

CITATION LIST

PATENT LITERATURE

PATENT LITERATURE 1: JP 3158894 U
PATENT LITERATURE 2: JP 2785049 B2

SUMMARY OF INVENTION

TECHNICAL PROBLEM

However, for an elderly person or a patient who has a limited range of joint motion, a tablet is desired which can be pinched more easily, has a shape hard to roll even if it drops from the hand, and also consider easiness of swallowing (swallowing).

Specifically, in the tablet described in Patent Literature 1, although the tablet has a shape difficult to roll due to the provision of the convex portion, further devising and improvement of the easiness of pinching have been required. In addition, swallowing may be impaired by the convex portions .

In the tablet described in Patent Literature 2, although it is difficult to roll by notching a part of the tablet body portion to form an inclined surface, and swallowing is also taken into consideration, devising and improvement were required again for easiness of pinching.

The present invention has been made in view of the above problems, and an object of the present invention is to provide a tablet which can be easily pinched even if an elderly person or a patient who has a limited range of joint motion is a target, and can be hard to roll even when slipping out of the hand.

Another object of the present invention is to provide a tablet taking the swallowing into consideration, even if the target is an elderly person or a rheumatoid arthritis patient complicated with Sjogren's syndrome, as an oral administration tablet.

SOLUTION TO PROBLEM

According to the tablet of the present invention, represented by claim 1, the above-mentioned problem is solved by a tablet which includes a tablet body portion having a three-dimensional shape; and a protruding portion continuously protruding from a side surface of the tablet body portion to the outside in an orthogonal direction orthogonal to a height direction of the tablet body portion, wherein the protruding portion has a first protruding portion and a second protruding portion protruding in opposite directions from each other via the tablet body portion, and the first protruding portion and the second protruding portion are disposed at different height positions from each other.

With the above configuration, it is possible to achieve a tablet including a universal design which can be easily pinched even if an elderly person or a patient who has a limited range of joint motion is a target, and is hard to roll even when slipping out of the hand.

Specifically, since the protruding portion protruding outward in the direction perpendicular to the height direction of the tablet body portion continuously from the side surface of the tablet body portion is provided, when the tablet is pinched, by pushing the protruding portion of the tablet (one end portion) from above, the protruding portion (the other end portion) on the opposite side of the tablet rises, and it is possible to form a space between the other end portion and an installation surface (the surface of the table or the storage surface of the tablet case). As a result, it is possible to greatly improve easiness of pinching.

Further, the protruding portion has a first protruding portion and a second protruding portion protruding in opposite directions from each other via the tablet body portion, and the first protruding portion and the second protruding portion are located at different height positions from each other. Accordingly, even if the upper surface of the tablet is placed in an upward position, or even if the upper surface is placed in a downward turned-over position, it is possible to secure the easiness of pinching at both positions.

In addition, since the protruding portions are provided, the shape is hard to roll even when slipping out of the hand, as compared with conventional round type or oval type tablets.

At this time, the first protruding portion protrudes from an upper portion of the side surface of the tablet body portion, the second protruding portion protrudes from a lower portion of the side surface of the tablet body portion, and the first protruding portion and the second protruding portion are formed to have a narrower width as the first and the second protruding portions are away from the tablet body portion.

As described above, since the first protruding portion and the second protruding portion each protrude from the upper portion and the lower portion of the side surface of the tablet body portion, the easiness of pinching can be further improved.

Specifically, when the upper surface of the tablet is placed at the upward position, the tablet can be easily raised by pushing the first protruding portion from above, and when the tablet is located at the turned-over position, the tablet can be easily raised, by pushing the second protruding portion from above.

Further, as described above, since the first protruding portion and the second protruding portion are formed to have a narrower width as they are away from the tablet body portion, in the case of tablets for oral administration, even if an elderly person or a rheumatoid arthritis patient complicated with Sjogren's syndrome is a target, it is expected that the tablets have a shape considering the swallowing.

At this time, the first protruding portion is formed such that the upper surface of the first protruding portion is substantially flush with the upper surface of the tablet body portion, and the second protruding portion is formed such that a bottom surface of the second protruding portion is substantially flush with a bottom surface of the tablet body portion.

With the above configuration, since the contact area between the tablet and the installation surface (the surface of the table or the storage surface of the tablet case) can be enlarged, the tablet is stabilized in the installed state, and as a result, it becomes easier to pinch the tablet, and becomes harder to roll.

At this time, the tablet may be for oral administration, the first protruding portion may have a first inclined surface which connects an end portion of the bottom surface of the tablet body portion on the first protruding portion side and a leading end portion of the first protruding portion, and which extends in a direction intersecting the bottom surface of the tablet body portion, and the second protruding portion may have a second inclined surface which connects the end portion of the upper surface of the tablet body portion on the second protruding portion side and a leading end portion of the second protruding portion, and which extends in a direction intersecting the upper surface of the tablet body portion.

As described above, since the tablet has the first inclined surface and the second inclined surface, when the tablet is inclined to pinch the tablet, it is possible to ensure a large contact area between the inclined surface of the tablet and the installation surface, the tablet is stabilized in a inclined state, and as a result, the tablet becomes easier to pinch.

Further, with the above configuration, since the protruding feeling of each protruding portion is improved and becomes smooth, it has a shape that takes into consideration the swallowing, as compared with a conventional tablet having a protruding portion (convex portion).

Especially, in the case of a rheumatoid arthritis patient, since there are many cases where a Sjogren's syndrome coexists, and many patients feel difficult for not only pinching but also swallowing, the effect based on the constitution of the present invention is expected to be more prominent.

At this time, when a length of the bottom surface of the tablet in the protruding direction of the protruding portion is set as L1 (mm) , and a length of the upper surface of the tablet in the protruding direction is set as L2 (mm) , and when an angle formed between an imaginary surface connecting an end portion of the bottom surface on the first protruding portion side and a leading end portion of the first protruding portion, and the bottom surface is set as $\theta_1$ (°), and an angle formed between an imaginary surface connecting an end portion of the upper surface on the second protruding portion side and a leading end portion of the second protruding portion, and the upper surface is set as $\theta_2$ (°) , at least one of the following formulas 1 and 2 may be satisfied:

$$\text{Formula 1: } L1 \times \sin(180° - \theta_1) \geq 3.4 \text{ (mm)}$$

$$\text{Formula 2: } L2 \times \sin(180° - \theta_2) \geq 3.4 \text{ (mm)}$$

With the above configuration, it is possible to achieve a tablet of a suitable shape, on the basis of the results of tests for evaluating the easiness of pinching of tablets, assuming an elderly person or a rheumatoid arthritis patient.

Specifically, as long as the height of the tablet can be ensured to be 3.4 mm or more when the end portion of the tablet is pushed from above and inclined, even in the case of an elderly person or a patient who has a limited range of joint motion, it is considered that the tablet can be stably pinched.

At this time, a value obtained by dividing the length L of the tablet by the width W of the tablet may be 1.79 or more and 3.72 or less.

According to the above configuration, it is possible to achieve a tablet having a suitable shape compatible with the easiness of pinching and swallowing, on the basis of the results of tests for evaluating the easiness of pinching and swallowing of a tablet, respectively.

ADVANTAGEOUS EFFECTS OF INVENTION

According to the tablet of the present invention, as a universal design, it is thought that it is possible to provide a tablet which can be easily pinched even if an elderly person or a patient who has a limited range of joint motion is a target, and is hard to roll even when slipping out of the hand.

Further, it is thought that it is possible to provide a tablet taking the swallowing into consideration, even if the target is an elderly person or a rheumatoid arthritis patient complicated with Sjogren's syndrome, as an oral administration tablet.

BRIEF DESCRIPTION OF DRAWINGS

FIG. 1 is a schematic diagram illustrating a shape of a tablet according to a first embodiment.
FIG. 2A is a side view of the tablet according to the first embodiment.
FIG. 2B is an upper surface view of the tablet according to the first embodiment.
FIG. 3 is a perspective view of the tablet according to the first embodiment.
FIG. 4A is a plan view of the tablet illustrated in FIG. 3.
FIG. 4B is a right side view of the tablet illustrated in FIG. 3.
FIG. 4C is a bottom view of the tablet illustrated in FIG. 3.
FIG. 4D is a left side view of the tablet illustrated in FIG. 3.
FIG. 4E is a rear view of the tablet illustrated in FIG. 3.
FIG. 4F is a front view of the tablet illustrated in FIG. 3.
FIG. 5 is a schematic view illustrating a shape of a tablet according to a second embodiment.
FIG. 6 is a perspective view of a tablet according to the second embodiment.

FIG. 7A is a plan view of the tablet illustrated in FIG. 6.
FIG. 7B is a right side view of the tablet illustrated in FIG. 6.
FIG. 7C is a bottom view of the tablet illustrated in FIG. 6.
FIG. 7D is a left side view of the tablet illustrated in FIG. 6.
FIG. 7E is a rear view of the tablet illustrated in FIG. 6.
FIG. 7F is a front view of the tablet illustrated in FIG. 6.
FIG. 8A is a view illustrating a state in which the tablet of the first embodiment is inclined.
FIG. 8B is a view illustrating a state in which the tablet of the second embodiment is inclined.
FIG. 9 is a diagram illustrating the details of a pinching easiness evaluation test of a tablet.
FIG. 10 is a graph showing the results of a pinching easiness evaluation test of a tablet using a round type tablet.
FIG. 11 is a graph showing the results of a pinching easiness evaluation test of a tablet using Examples 1 and 2.
FIG. 12 is a graph showing the results of a pinching easiness evaluation test of a tablet using Example 1.
FIG. 13 is a view illustrating details of a swallowing evaluation test of a tablet.
FIG. 14 is a graph showing the results of a swallowing evaluation test of a tablet using Examples 1 and 2.
FIG. 15 is a graph showing the results of a swallowing evaluation test of a tablet using Example 1.

DESCRIPTION OF EXAMPLES

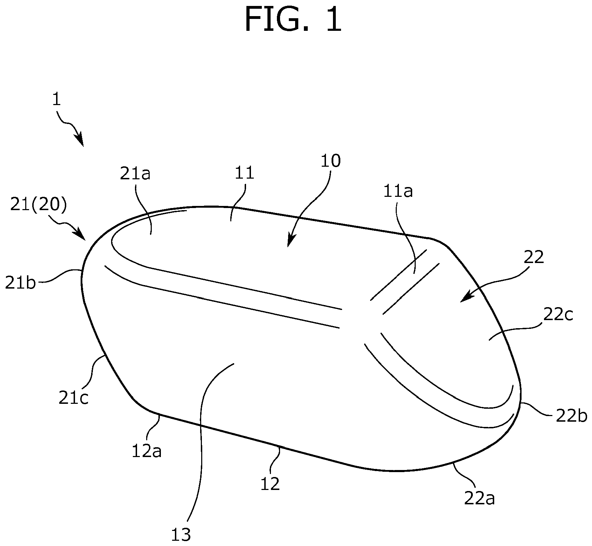

Hereinafter, embodiments of the present invention will be described with reference to FIGS. 1 to 15.

The present embodiment relates to the invention of a tablet. The tablet includes a tablet body portion having a three-dimensional shape, and a protruding portion that protrudes outward in a longitudinal direction of the tablet body portion continuously from a side surface of the tablet body portion. The protruding portion includes a first protruding portion and a second protruding portion that protrude in opposite directions from each other via the tablet body portion.

The first protruding portion and the second protruding portion protrude from an upper portion and a lower portion of the side surface of the tablet body portion, and are formed to become narrower as going away from the tablet body portion.

Hereinafter, the first embodiment and the second embodiment will be exemplified, but the present invention is not limited thereto.

<First Embodiment of Tablet>

A tablet 1 of the first embodiment is a pharmaceutical tablet for oral administration (for example, a tablet, an orally disintegrating tablet), and is usually stored in a bottle or a press-through package (PTP) packaging body, transported and managed.

The tablet 1 is not particularly limited to a tablet for oral administration, can be various changed, and may be, for example, a tablet for oral use (lozenge tablet), an external use tablet, or the like. Also, it is not particularly limited to pharmaceutical tablets, and may be for health foods (supplements) or sweets. Also, it may be a pharmaceutical tablet having a dividable line that can be divided into a plurality of portions.

The tablet 1 can be manufactured by a known method. For example, it can be manufactured using a 3D printer. As a lamination method of a 3D printer, for example, it is possible to adopt a fused deposition modeling method in which a thermoplastic resin to be a three-dimensional molding material is thermally melted, extruded from a nozzle, and molded while being laminated on a molding stage, or a powder bed printing method that spreads powder resin as a three-dimensional molding material on the molding stage and sprays adhesive materials. Besides that, it is also possible to adopt a stereolithography, a powder selective laser sintering method or the like.

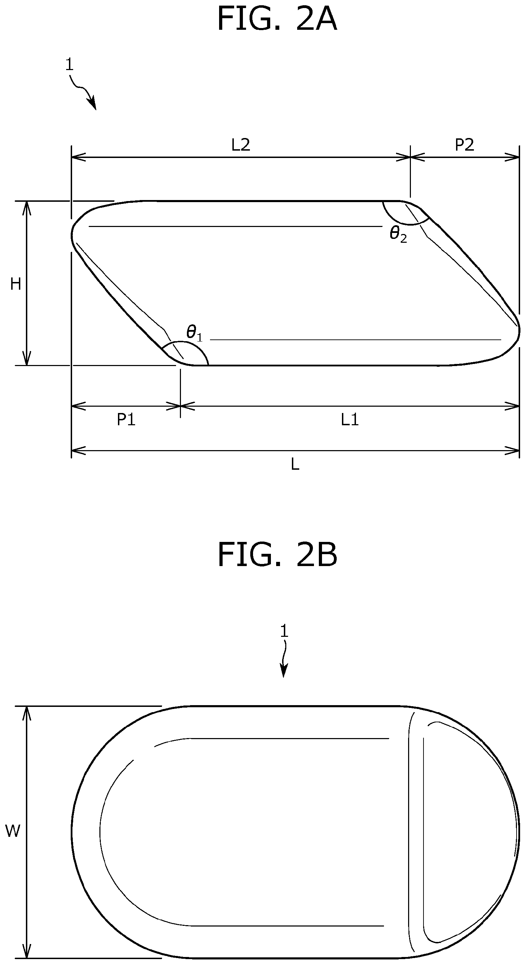

As illustrated in FIGS. 1, 2A, and 2B which are schematic diagrams illustrating the shape, the tablet 1 is formed so that a longitudinal sectional shape is substantially a parallelogram, and a length (major axis) L of the tablet is 10.0 to 11.6 mm (desirably 10.4 mm), a width W (minor axis) is 4.9 to 5.6 mm (desirably 5.4 mm), a height H is 3.0 to 4.0 mm (desirably 3.6 mm) , and a protruding length P is 1.0 to 3.0 mm (desirably 2.0 mm) .

Further, the volume of the tablet 1 is 125 to 165 mm$^3$ (desirably 146 mm$^3$), and the weight is 165 to 205 mg (desirably 187 mg) .

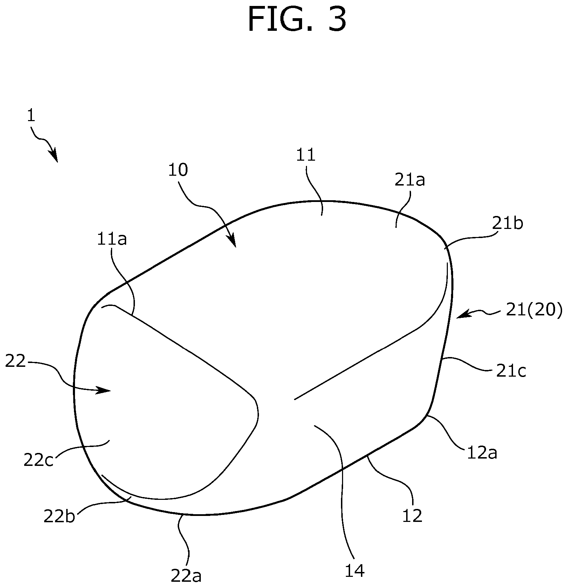

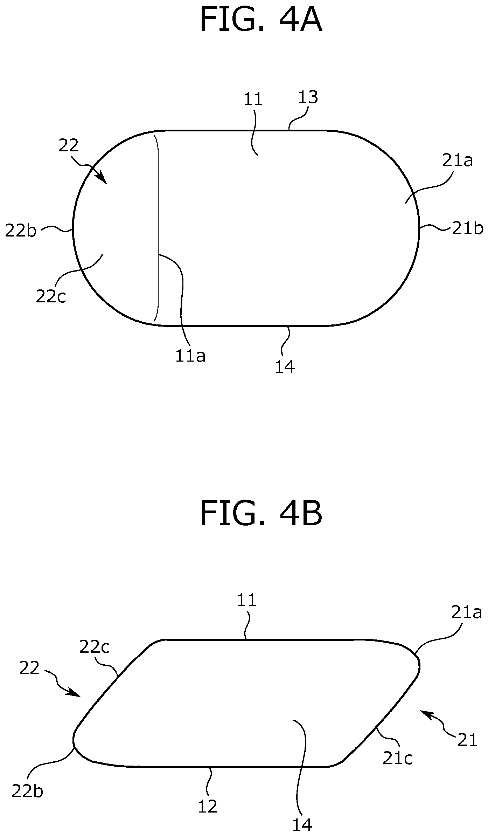

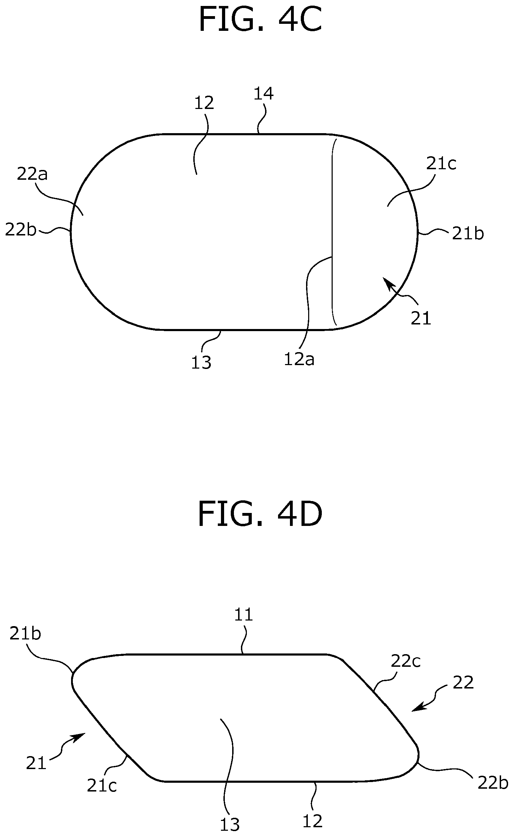

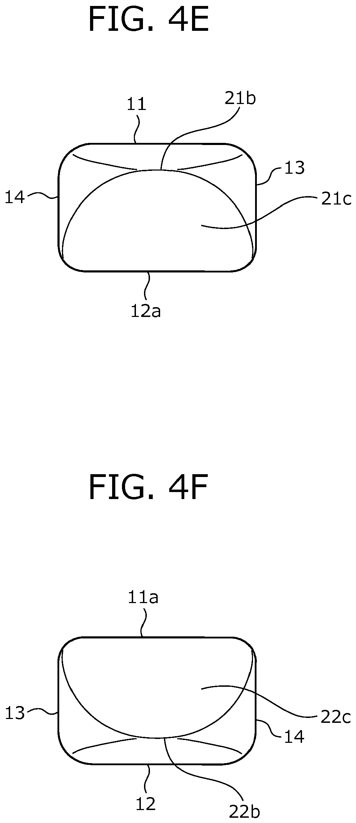

FIG. 3 is a perspective view of the tablet 1, FIG. 4A is a plan view of the tablet 1 illustrated in FIG. 3, FIG. 4B is a right side view of the tablet 1 illustrated in FIG. 3, FIG. 4C is a bottom view of the tablet 1 illustrated in FIG. 3, FIG. 4D is a left side view of the tablet 1 illustrated in FIG. 3, FIG. 4E is a rear view of the tablet 1 illustrated in FIG. 3, and FIG. 4F is a front view of the tablet 1 illustrated in FIG. 3.

As illustrated in FIGS. 1, 3, and 4A to 4F, the tablet 1 mainly includes a tablet body portion 10 having a three-dimensional shape, and a protruding portion 20 that continuously protrudes to the outside in a longitudinal direction of the tablet body portion 10 from the side surfaces 13 and 14 of the tablet body portion 10.

The tablet body portion 10 is formed of a rectangular body, and its upper surface and bottom surface are configured to form a flat surface.

The protruding portion 20 has a first protruding portion 21 and a second protruding portion 22 protruding in opposite directions from each other via the tablet body portion 10.

The side surfaces 13 and 14 are outer surfaces in a width direction (minor axis direction) of the tablet body portion 10.

The protruding portions of each of the first protruding portion 21 and the second protruding portion 22 are disposed at different height positions from each other, and in detail, the first protruding portion 21 protrudes from the upper portion of the side surfaces 13 and 14 of the tablet body portion 10, and the second protruding portion 22 protrudes from the lower portion (a lower end portion) of the side surfaces 13 and 14 of the tablet body portion 10. Further, the first protruding portion 21 and the second protruding portion 22 are formed to become narrower as they are away from the tablet body portion 10.

The first protruding portion 21 is formed such that an upper surface 21a thereof is substantially flush with the upper surface 11 of the tablet body portion 10, and the second protruding portion 22 is formed so that a bottom surface 22a thereof is substantially flush with the bottom surface 12 of the tablet body portion 10.

The first protruding portion 21 has a first inclined surface 21c connecting an end portion 12a of the bottom surface 12 of the tablet body portion 10 on the first protruding portion 21 side and a leading end portion 21b of the first protruding portion 21, and extending in a direction intersecting with the bottom surface 12 of the tablet body portion 10.

Note that a first inclined angle $\theta_1$ (°), which is an angle between the first inclined surface 21c and the bottom surface 12 of the tablet 1, is 120 to 150° (desirably 135°) .

Further, the second protruding portion 22 has a second inclined surface 22c connecting an end portion 11a of the upper surface 11 of the tablet body portion 10 on the second protruding portion 22 side and a leading end portion 22b of the second protruding portion 22, and extending in a direction intersecting the upper surface 11 of the tablet body portion 10.

Note that a second inclined angle $\theta_2$ (°), which is an angle between the second inclined surface 22c and the upper surface 11 of the tablet 1, is 120 to 150° (desirably 135°) .

In the above configuration, as illustrated in FIGS. 1, 2A and 2B, a corner portion of the entire tablet 1 is curved by being rounded and chamfered. Therefore, the corner portion of the entire tablet 1 becomes smooth to have a shape in which swallowing is taken into consideration.

In particular, by forming the corner portion of the protruding portion 20 of the tablet 1 so as to be curved, it is considered that swallowing can be improved even for an elderly person or a rheumatic patient.

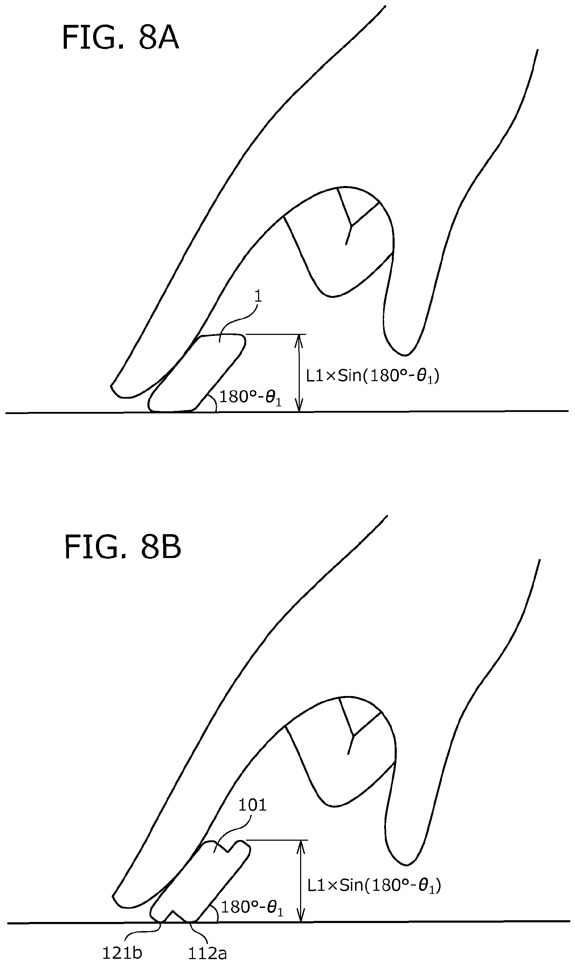

Next, a method of pinch the tablet 1 from a bottle or a PTP packaging body and pinching it will be described with reference to FIG. 8A.

First, with respect to the tablet 1 placed on a predetermined installation surface (for example, the front surface of the table), the upper surface of the first protruding portion 21 is pushed from above with the forefinger. In doing so, the tablet 1 is inclined toward the first protruding portion 21 side, the second protruding portion 22 rises, and a space can be formed between the second protruding portion 22 and the installation surface.

Further, in the state of pressing the upper surface of the tablet 1 with the forefinger, the bottom surface of the tablet 1 is pressed with thumb using the above space. By doing so, the tablet 1 can be pinched, while being pinched between the forefinger and the thumb.

As illustrated in FIG. 8A, a height (mm) when the tablet 1 is inclined is obtained by the following formula, when the length of the bottom surface of the tablet 1 is set as L1 (mm) and the inclined angle formed between the bottom surface and the inclined surface is set as $\theta_1$ (°).

$$\text{Height when tablet 1 is inclined} = L1 \times \sin(180° - \theta_1)$$

By the above method, easiness of pinching of the tablet 1 can be greatly improved.

In particular, since the tablet 1 can be inclined with a simple operation and the height of the tablet to such an extent that it is easy to pinch with the fingers can be secured, it is thought that it is possible to easily pinch the tablet, even when the elderly person or a patient who has a limited range of joint motion is a target.

According to the above method, the bottom surface 12 of the tablet body portion 10 and the bottom surface 22a of the second protruding portion 22 form a continuous plane, and a contact area between the bottom surface of the tablet 1 and the installation surface can be increased. Accordingly, the tablet 1 in the placed state is stabilized, and a result, it is easy to incline the tablet 1.

Since the tablet 1 has the first inclined surface 21c (the second inclined surface 22c), when the tablet 1 is inclined, a large contact area between the first inclined surface 21c of the tablet 1 and the installation surface can be secured. Accordingly, the tablet 1 in an inclined state is stabilized, and as a result, the tablet 1 can be easily pinched.

<Second Embodiment of Tablet>

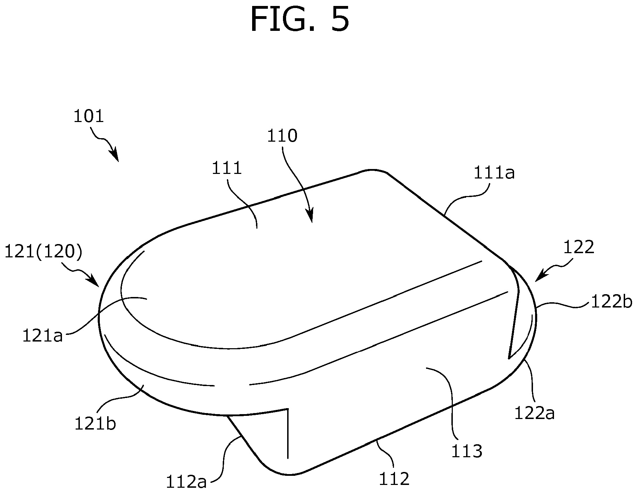

Next, the second embodiment of the tablet will be described with reference to FIGS. 5, 6, 7A to 7F, and 8B. The repeated description of tablet 1 will not be described.

In the tablet 101 of the second embodiment, as illustrated in FIG. 5, which is a schematic diagram illustrating the shape, the shape of the protruding portion 120 protruding from the side surface of the tablet body portion 110 is different from that of the tablet 1.

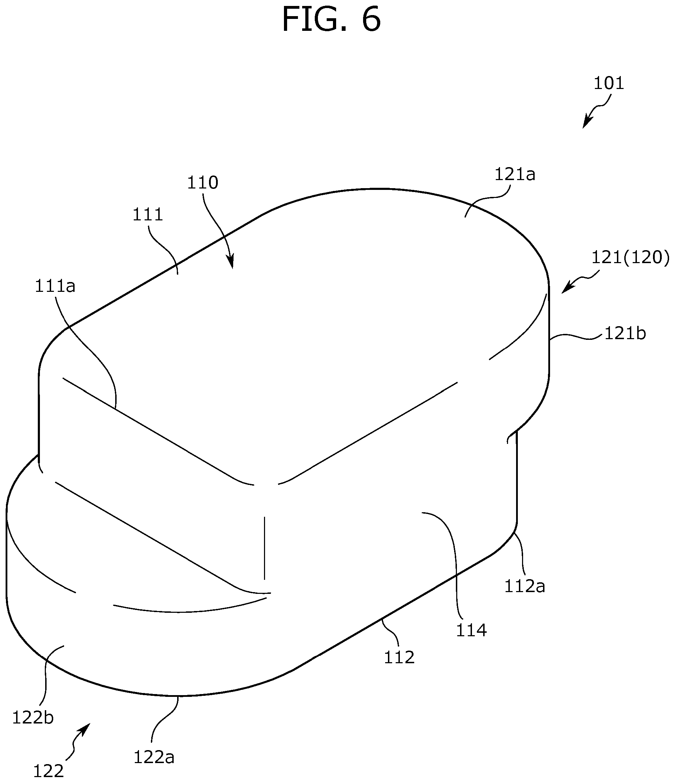

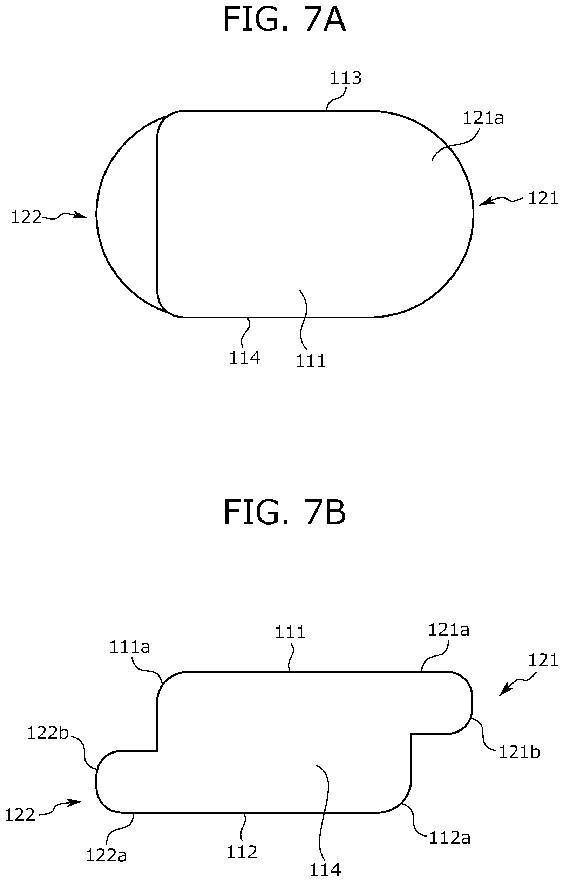

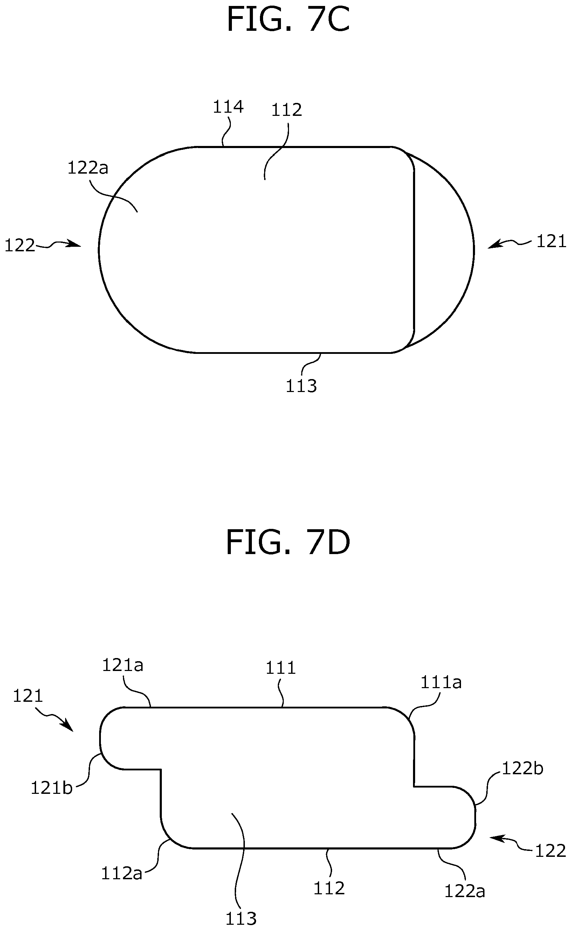

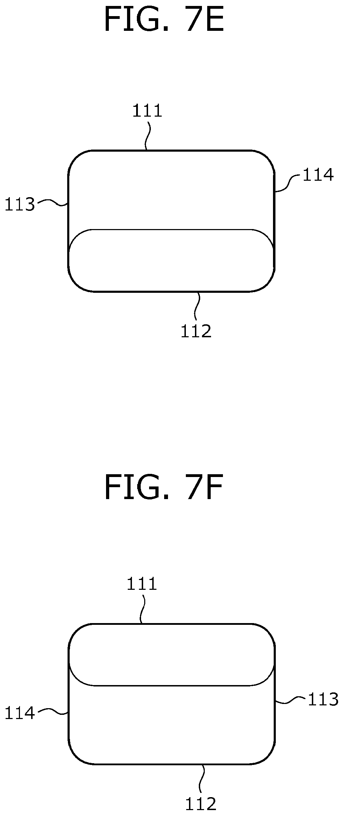

FIG. 6 is a perspective view of the tablet 101, FIG. 7A is a plan view of the tablet 101 illustrated in FIG. 6, FIG. 7B is a right side view of the tablet 101 illustrated in FIG. 6, FIG. 7C is a bottom view of the tablet 101 illustrated in FIG. 6, FIG. 7D is a left side view of the tablet 101 illustrated in FIG. 6, FIG. 7E is a rear view of the tablet 101 illustrated in FIG. 6, and FIG. 7F is a front view of the tablet 101 illustrated in FIG. 6. As illustrated in FIGS. 5, 6 and 7A to 7F, the protruding portion 120 has a first protruding portion 121 and a second protruding portion 122 protruding in opposite directions from each other via the tablet body portion 110, and the protruding portions 121 and 122 are formed to become narrower as they are away from the tablet body portion 110.

The first protruding portion 121 protrudes from the upper end portion of the side surfaces 113 and 114 of the tablet body portion 110, and the upper surface 121a thereof is formed to be substantially flush with the upper surface 111 of the tablet body portion 110.

The second protruding portion 122 protrudes from the lower end portion of the side surfaces 113 and 114 of the tablet body portion 110, and the second protruding portion 122 is formed such that the bottom surface 122a thereof is substantially flush with the bottom surface 112 of the tablet body portion 110.

A first inclined angle $\theta_1$ (°), which is an angle between an imaginary surface connecting the end portion 112a of the bottom surface 112 on the first protruding portion 121 side and the leading end portion 121b of the first protruding portion 121, and the bottom surface 112 of the tablet body portion 110, is 120 to 150° (desirably 135°) .

Further, a second inclined angle $\theta_2$ (°), which is an angle between an imaginary surface connecting the end portion 111a of the upper surface 111 on the second protruding portion 122 side and the leading end portion 122b of the second protruding portion 122, and the upper surface 111 of the tablet body portion 110, is 120 to 150° (desirably 135°).

The bottom surface of the first protruding portion 121 and the side surfaces 113 and 114 of the tablet body portion 110 extend in a planar form in a positional relation orthogonal (intersecting) with each other. Also, the upper surface of the second protruding portion 122 and the side surfaces 113 and 114 of the tablet body portion 110 also extend in a planar form in a positional relation orthogonal (intersecting) with each other.

With the above configuration, as compared with the tablet 1, a larger space is secured in the lower portion of the first protruding portion 121 and the upper portion of the second protruding portion 122 in the tablet 101.

Therefore, by utilizing the space, it is possible to pinch the first protruding portion 121 or the second protruding portion 122 as it is, for example, without inclining the tablet 101.

Even with the shape of the tablet 101 having the above-described configuration, it is possible to greatly improve easiness of pinching in the same manner as the tablet 1.

In particular, as illustrated in FIG. 8B, since the tablet 101 can be inclined with a simple operation and the height of the tablet to such an extent that it is easy to pinch with fingers can be secured, it is thought that it is possible to easily pinch the tablet, even when the elderly person or the patient who has a limited range of joint motion is a target.

Further, as illustrated in FIG. 8B, the height (mm) when the tablet 101 is inclined is obtained by the following formula, when the length of the bottom surface of the tablet 101 is set as L1 (mm), and an inclined angle formed by an imaginary surface connecting the end portion 112a of the bottom surface 112 on the first protruding portion 121 side and the leading end portion 121b of the first protruding portion 121 with the bottom surface 112 is set as $\theta_1$ (°).

$$\text{Height when tablet 101 is inclined} = L1 \times \sin(180° - \theta_1)$$

<Other Embodiments>

In the above embodiment, as illustrated in FIG. 1, the protruding portion 20 protrudes outward in the longitudinal direction of the tablet 1 continuously from the side surface of the tablet body portion 10, but can be changed without any particular limitation, and may protrude to the outside in the direction orthogonal (intersecting) with the height direction of the tablet 1.

For example, the protruding portion 20 may protrude outward in the width direction of the tablet 1.

In the above embodiment, as illustrated in FIG. 1, the side surface of the tablet body portion 10 is substantially parallel but can be changed without any particular limitation, and the side surface of the tablet 1 may be in the form having a curvature toward the outside.

In the above embodiment, as illustrated in FIGS. 1 and 5, the first protruding portions 21 and 121 and the second protruding portions 22 and 122 protrude from the upper portion and the lower portion of the side surface of the tablet body portions 10 and 110, respectively. However, it can be changed without any particular limitation, and they may be disposed at different height positions from each other.

In the above embodiment, as illustrated in FIG. 1, the first protruding portion 21 and the second protruding portion 22 are formed so as to become narrower as they are away from the tablet body portion 10, but they can be changed without any particular limitation.

For example, the first protruding portion 21 and the second protruding portion 22 may be formed to have substantially the same width.

In the present embodiment, the tablet according to the present invention has mainly been described.

Example

<Example 1>

Hereinafter, examples of the present invention will be described in detail. It should be noted that the present invention is not limited to the examples.

A first example of the tablet was manufactured, using a PLA filament (3D Printer Filament PLA 1.75 mm Violet, manufactured by Zhuhai CTC Electronic Co., Ltd.) which is a printing base material of a 3D printer, and a 3D printer (Eagleed, manufactured by Reis Enterprise Co., Ltd.).

As illustrated in FIG. 5, the shape of the first example has the same shape as the tablet 101 of the present embodiment, and has a tablet length L = 10.4 mm, a width W = 5.4 mm, a height H = 3.8 mm, a protruding length P = 2.0 mm, an inclined angle $\theta$ = 135°, a height when the tablet is inclined = 5.9 mm, a volume 147 mm$^3$, and a weight 177 mg.

<Example 2>

A second example of tablet was manufactured, using the PLA filament and a 3D printer used in Example 1.

As illustrated in FIG. 1, the shape of the second example is the same shape as the tablet 1 of the present embodiment, and has a tablet length L = 10.4 mm, a width W = 5.6 mm, a height H = 3.6 mm, a protruding length P = 2.5 mm, an inclined angle $\theta$ = 125°, a height when the tablet is inclined = 6.5 mm, a volume 146 mm$^3$, and a weight 187 mg.

<Test Example 1: Pinching Easiness Evaluation Test 1 of Tablets>

Tests were carried out to check the height of the easiness of pinching of the tablets, using round type tablets with different heights.

At the time of the test, rheumatoid arthritis was simulated in a rheumatism patient who has a limited range of joint motion. Specifically, a right hand joint fixture (12349-070 (PK1), manufactured by Kyoto Kagaku Co., Ltd.) of the rheumatoid arthritis (7), and a right hand portion of Urashima Taro-Elderly Person Experience Program (Wonderful Aging Club, Inc.) are worn on the subject's right hand.

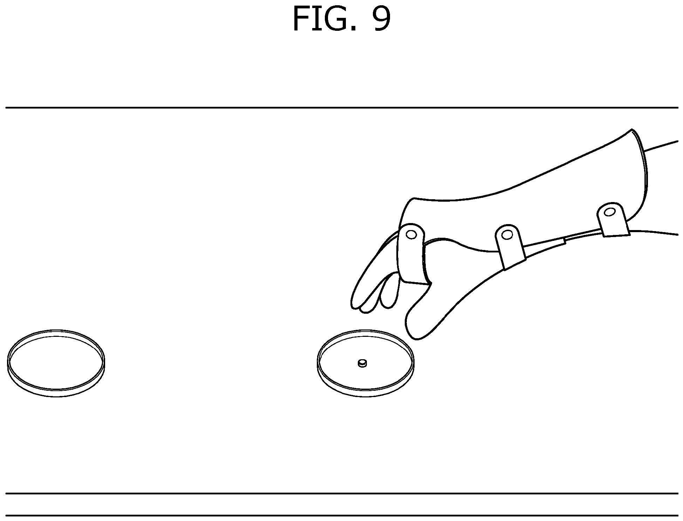

Specifically, as illustrated in FIG. 9, a tablet placed on a petri dish was pinched, and a tablet in a pinched state was moved to another petri dish separated apart by about 15 cm from the petri dish. Further, an operation of pinching the moved tablets again and returning to the original petri dish was performed. At the beginning of the measurement, an operation of placing the right hand on his own knee and returning the right hand to his knee every time pinching once was performed.

The operation was executed five times, and the operation time required for five round trips was measured.

As a sample of the tablet, as shown in the following Table 1, round type tablets (about 8 mm in diameter) with different height of tablets were prepared. In other words, a total of 10 samples having a height of tablet of 0.5 to 7.4 mm were prepared.

Three subjects were used, three times per type of sample.

[Table 1]

| Sample | Height (mm) of tablet |
|--------|-----------------------|
| A | 0.5 |
| B | 1.3 |
| C | 2.1 |
| D | 3.4 |

(continued)

| Sample | Height (mm) of tablet |
| --- | --- |
| E | 4.0 |
| F | 4.4 |
| G | 4.8 |
| H | 5.8 |
| I | 6.1 |
| J | 7.4 |

(Results and Examination of Test Example 1)

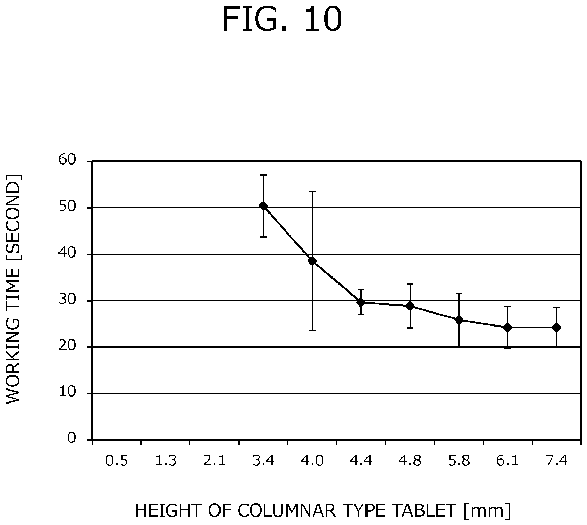

A graph showing a relation between the height of tablet and working time by analyzing the above test results is shown in FIG. 10.

Samples A to C having height of tablet of 0.5 to 2.1 mm could not be pinched. It was possible to securely pinch samples D to J having the height of tablet of 3.4 to 7.4 mm at the working time of 50.4 seconds, 38.6 seconds, 29.7 seconds, 28.9 seconds, 25.9 seconds, 24.2 seconds and 24.2 seconds in order.

Sample E having a height of tablet of 4.0 mm showed a significant improvement in working time ($P < 0.05$, one side paired t test), as compared to sample D with a height of 3.4 mm which was able to pinch.

Furthermore, Sample F with a height of tablet of 4.4 mm was significantly shorter in working time ($P < 0.05$, one side paired t test) than Sample E with a height of 4.0 mm.

From the results in Test Example 1, it is suggested that, in order for a rheumatoid arthritis patient to stably pinch a round type tablet, it is desirable that the height of the tablet be 3.4 mm or higher, and in order to easily pinch the tablets, it is desirable that the height of the tablet be 4.0 mm or higher.

Further, it is suggested that the height of the tablet is desirably 4.4 mm or higher in order to further facilitate pinching of the tablet.

<Test Example 2: Pinching Easiness Evaluation Test 2 of Tablets>

A test of checking the easiness of pinching of the tablet of the present embodiment was conducted, using the tablets of Examples 1 and 2 in the same manner as in Test Example 1.

As a comparative object, a conventional round tablet (a diameter 7.6 mm, a height 3.5 mm, a radius of curvature 11.3 R, a volume 128 mm$^3$, a weight 169 mg, hereinafter sometimes abbreviated as an conventional tablet) was prepared. The conventional tablet was prepared by compacting one obtained by mixing and homogenizing additives such as excipients.

For the tablets of Examples 1 and 2, as illustrated in FIGS. 8A and 8B, the tablets were inclined so as to be pinched.

There were three subjects, and the test was performed three times per type of sample.

(Results and Examination of Test Example 2)

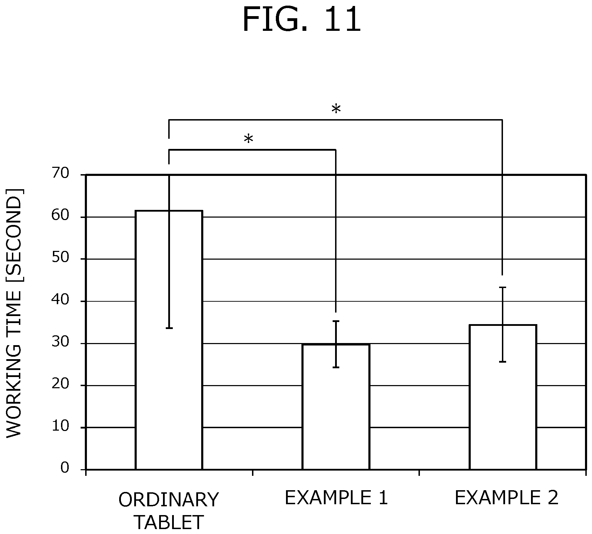

FIG. 11 shows a graph showing a relation between the conventional tablet, the tablets of Examples 1 and 2 and the working time by analyzing the above test results.

The working time of the conventional tablet was 61.4 seconds, and the working time of Examples 1 and 2 were 29.8 seconds and 34.4 seconds in order.

Multiple comparison tests were performed for each index ($P < 0.05$, Holm method, one side paired t test) . The p values of Examples 1 and 2 were 0.00456, and 0.00373 in order.

The working time of the tablets of Examples 1 and 2 was significantly shorter than that of the conventional tablets.

Incidentally, the Holm method was analyzed on the basis of the reference (Holm S. A simple sequentially rejective multipletest procedure. Scand J Stat. 1979; 6: 65-70).

From the results of Test Example 2, it was suggested that as long as the shape of Example 1 or 2 is used, even if the patient who has a limited range of joint motion is a target, it is possible to easily pinch the tablet with a simple operation of inclining and pinching the tablet.

Further, from the results of Test Examples 1 and 2, it was found that it is desirable to set the height of the tablet to 3.4 mm or more when these tablets are inclined in order to facilitate pinching of the tablets 1 and tablets 101.

In other words, it was found that, when the length of the bottom surface of the tablet is set as L1 (mm), and an angle formed between the imaginary surface connecting the end portion side of the bottom surface on the first protruding portion side and the leading end portion of the first protruding portion, and the bottom surface is set as $\theta_1$ (°), it is desirable to satisfy the following formula 1.

$$\text{Formula 1: } L1 \times \sin(180° - \theta_1) \geq 3.4$$

Incidentally, even when the tablet is placed in a position at which the tablet is turned upside down, the same is true.

That is, when the length of the upper surface of the tablet is set as L2 (mm), and an angle formed between the imaginary surface connecting the end portion of the upper surface on the second protruding portion side and the leading end portion of the second protruding portion, and the upper surface is set as $\theta_2$ (°), it was found that it is desirable to satisfy the following formula 2.

$$\text{Formula 2: } L2 \times \sin(180° - \theta_2) \geq 3.4$$

<Test Example 3: Pinching Easiness Evaluation Test 3 of Tablets>

A test of evaluating a preferable shape for making the tablet of the present embodiment easy to pinch was performed, using a tablet having the same shape as in Example 1, by the same method as in Test Example 1.

As a sample of tablets, as shown in the following Table 2, tablets each having a length L (mm) and a width W (mm) changed were prepared.

At that time, the height H of the tablet was set to 3.5 mm, the protruding length P was set to 2.0 mm, and the inclined angle θ was set as 135°. In actual measurement, the height H of the tablet was 3.7 to 3.8 mm, and the protruding length P was 2.0 to 2.1 mm.

[Table 2]

| Sample | Length L (mm) | width W (mm) | Length L/width W | Height (mm) when inclining tablet |
|---|---|---|---|---|
| 1 | 10.0 | 5.6 | 1.79 | 5.7 |
| 2 | 10.4 | 5.4 | 1.93 | 5.9 |
| 3 | 10.9 | 5.0 | 2.18 | 6.2 |
| 4 | 11.6 | 4.9 | 2.37 | 6.8 |
| 5 | 13.0 | 4.4 | 2.95 | 7.8 |
| 6 | 14.5 | 3.9 | 3.72 | 8.8 |
| 7 | 19.2 | 2.7 | 7.11 | 12.2 |
| 8 | 28.9 | 1.7 | 17.0 | 19.0 |

The height H of the tablet was set to 3.5 mm in accordance with to the height of conventional tablets.

When the protruding length P of the tablet exceeds 2 mm, there is risk of inferiority of the stability when the tablet is settled, whereas when the protruding length P is less than 2 mm, there is a risk of difficulty in inclining the tablet. Accordingly, the protruding length P was set as 2.0 mm.

The inclined angle θ of the tablet was set to 135° in consideration of the balance between when the tablet was settled and when the tablet was inclined.

The height when the tablet was inclined was calculated from the following formula.

$$\text{Height when tablet is inclined} = (\text{length L} - \text{protruding length P}) \times \sin(180° - 135°)$$

As a comparative target, an conventional tablet of a general round type (a diameter 7.6 mm, a height 3.5 mm, a radius of curvature 11.3 R, a volume 128 mm$^3$, and a weight 169 mg) was prepared in the same manner as in Test Example 2.

For the tablets having the same shape as in Example 1, as illustrated in FIG. 8B, the tablets were inclined and pinched.

There were three subjects, and the test was performed three times per type of sample.

(Results and Examination of Test Example 3)

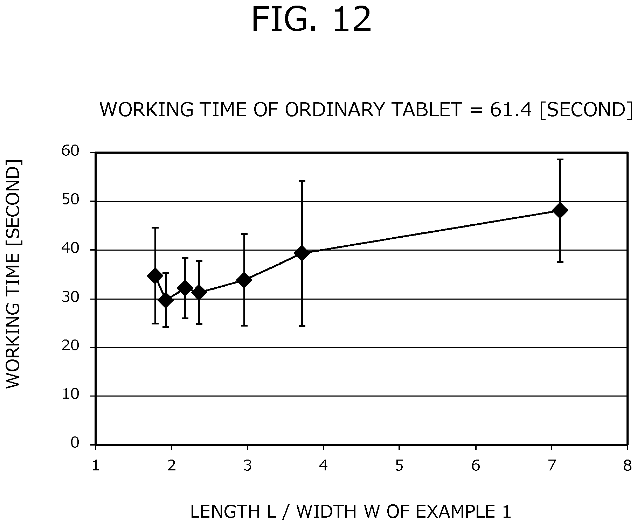

FIG. 12 shows a graph showing a relation between the "length L/width W" of a tablet having the same shape as in Example 1 and the working time by analyzing the above test results.

Sample 8 could not be pinched because the width W of the tablet was small. Further, for samples 1 to 7, the working times were 34.8 seconds, 29.8 seconds, 32.2 seconds, 31.3 seconds, 33 . 9 seconds, 39. 3 seconds, and 48.1 seconds in order. Further, the working time of ordinary tablets was 61.4 seconds.

Multiple comparison tests were performed for each index ($P < 0.05$, Holm method, one side paired t test) . The p values of Samples 1 to 7 were 0.0121, 0.0114, 0.00478, 0.00926, 0.0135, 0.00717, and 0.0694 in order.

The working time of the tablets of Samples 1 to 6 was significantly shorter than that of conventional tablets. Also, among the tablets of Samples 1 to 6, the working time of the tablets of Sample 2 was the shortest.

From the results of Test Example 3, it was suggested that the width W of the tablet is preferably set to 3.9 mm or more in order for the patient who has a limited range of joint motion to easily pinch the tablet 101 having the same shape as in Example 1. When the width of the tablet was set to less than 3.9 mm, it was found that the tablet unstably rolled when raising the protruding portion and it was difficult to pinch. Further, since the mechanism for facilitating the pinching is the same, it is thought that the same also applies to the tablets having the same shape as in Example 1.

That is, it was suggested that as long as the tablet has the same shape as in Example 1 in which the width W of the tablet is set to 3.9 mm or more, even if the patient who has a limited range of joint motion is a target, it is possible to easily pinch the tablet with a simple operation of inclining and pinching the tablet.

Further, the tablet having the same shape as in Example 1 has a higher height when the tablet is inclined as the length L of the tablet becomes longer. However, since the stability of the tablet is impaired when the tablet is settled and inclined by the shortage of the width W, it was found that it became more difficult to pinch the tablet.

In addition, the height when the tablet is inclined decreases as the length L of the tablet decreases. However, since the stability of the tablet is improved when the tablet is settled and inclined by the increase of the width W, it was found that it became easier to pinch the tablets.

That is, it was found that, in the shape of a tablet having the same shape as in Example 1, it is preferable that the value of the preferable length L/width W be 1.79 or more and 3.72 or less.

Further, in the case of a shape of Sample 2 (a tablet length L = 10.4 mm, a width W = 5.4 mm, a height H = 3.8 mm, a protruding length P = 2.0 mm, an inclined angle $\theta$ = 135°, and a height when the tablet is inclined = 5.9 mm) having the same shape as in Example 1, it was suggested that tablets are easiest to pinch for patient who has a limited range of joint motion.

<Test Example 4: Swallowing Evaluation Test 1 of Tablet>

A test of checking the swallowing of the tablets was performed, using the tablets having the same shape as in Examples 1 and 2.

This test was conducted with reference to the test method (evaluation of slipperiness) described in JP 2013-46453 A1.

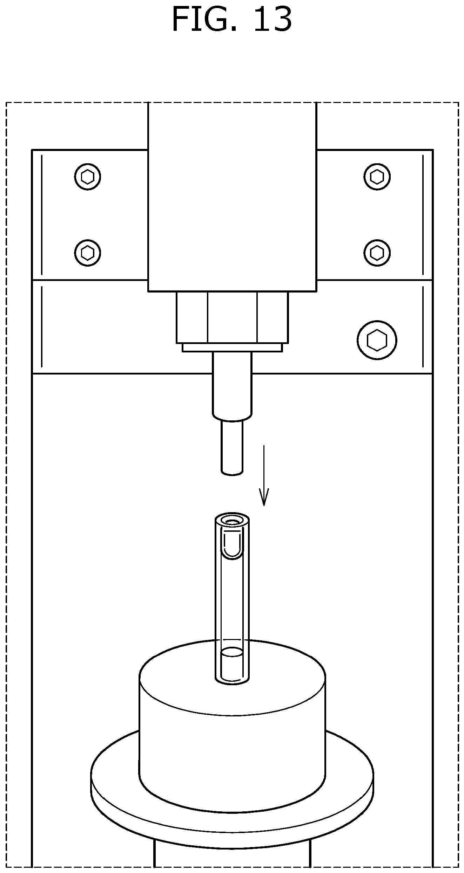

Specifically, as illustrated in FIG. 13, a silicon tube (6 $\times$ 12: an inner diameter 6 mm, and an outer diameter 12 mm) was cut to a length of 5 cm and set in a creep meter (RE2-33005 S, manufactured by YAMADEN co., ltd.) . The lower end was stoppered with absorbent cotton, and the tablet was filled from above.

When 1 mL of ethanol passed through the silicon tube with a syringe and ethanol was released, the probe set in the creep meter was immediately inserted into the silicon tube, and moved from the top to the bottom by 20 mm at a speed of 5 mm/sec, and the stress at that time was measured.

Incidentally, the stress was calculated from the measured load/plunger cross-sectional area (the cross-sectional area of the plunger was 19.6 $mm^2$).

As a comparative object, an conventional tablet of a general round type (a diameter 7.6 mm, a height 3.5 mm, a radius of curvature 11.3 R, a volume 128 $mm^3$, and a weight 169 mg) was prepared in the same manner as in Test Example 2.

The test was performed three times per type of sample.

Further, it was checked that stress values were equal between conventional tablets (using mannitol as the main base) manufactured by a general manufacturing method and conventional tablets (using PLA as the main base) manufactured using a 3D printer, and it was checked in advance that there was no significant difference due to the difference between these manufacturing methods and bases.

(Results and Examination of Test Example 4)

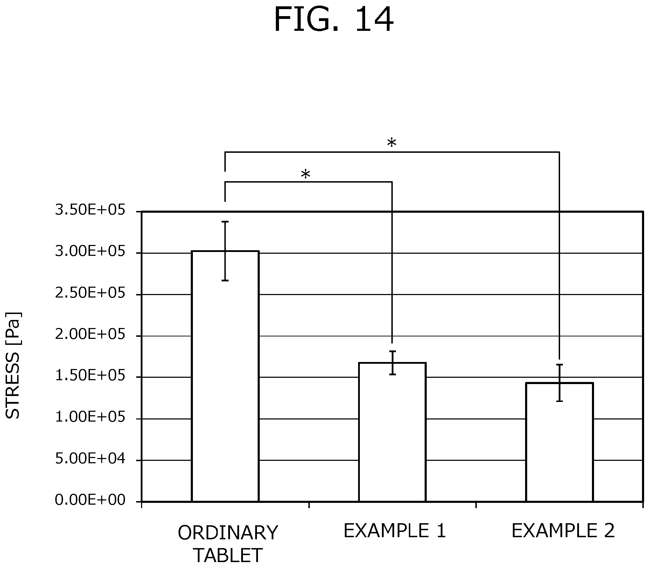

FIG. 14 shows a graph showing a relation between the conventional tablet, the tablets of Examples 1 and 2 and the stress by analyzing the above test results.

The stress of the conventional tablet was $3.03 \times 10^5$ Pa, and the stresses of Examples 1 and 2 were $1.67 \times 10^5$ Pa and $1.43 \times 10^5$ Pa in order.

Multiple comparison tests were conducted for each index ($P < 0.05$, Holm method, one side unpaired t test, F test was preliminarily conducted, t verification of Student was adopted in the case of equal variance, and t verification of Welch was adopted in the case of unequal variance). The p values of Examples 1 and 2 were 0.00178 and 0.00271 in order.

The tablets of Examples 1 and 2 had a significantly lower stress than the usual tablets, which was about half.

From the results of Test Example 4, it was suggested that the same shape as Tablet 1 and Tablet 101 as the tablet for oral administration would be a tablet with superior swallowing compared to conventional tablets.

<Test Example 5: Swallowing Evaluation Test 2 of Tablet>

A test of evaluating a preferable shape for ensuring the swallowing of the tablet of the present embodiment was performed, using a tablet having the same shape as in Example 1, by the same method as in Test Example 4.

As a sample of tablets, as shown in the above Table 2, tablets each having a length L (mm) and a width W (mm) changed were prepared.

At that time, the height H of the tablet was set to 3.5 mm, the protruding length P was set to 2.0 mm, and the inclined angle θ was set to 135°. In actual measurement, the height H was 3.7 to 3.8 mm, and the protruding length P was 2.0 to 2.1 mm.

As a comparative target, an conventional tablet of a general round type (a diameter 7.6 mm, a height 3.5 mm, a volume 128 mm$^3$, and a weight 169 mg) was prepared in the same manner as in Test Example 2.

The test was performed three times per type of sample.

(Results and Examination of Test Example 5)

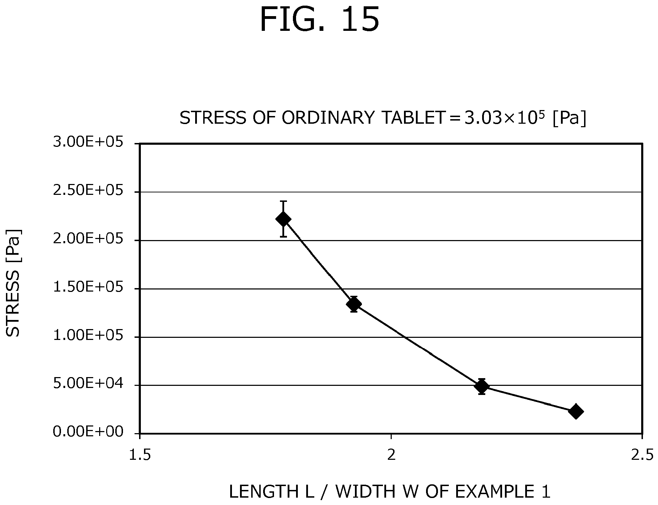

FIG. 15 shows a graph showing a relation between the "length L/width W" of a tablet having the same shape as in Example 1 and stress by analyzing the above test results.

The stress of the conventional tablet was $3.03 \times 10^5$ Pa.

The stress of Samples 1 to 4 was $2.22 \times 10^5$ Pa, $1.34 \times 10^5$ Pa, $4.87 \times 10^4$ Pa, and $2.29 \times 10^4$ Pa in order, and it was smaller than the stress of conventional tablets. For Samples 5 to 8, the width W of the tablet was small and it was not evaluated because it slipped through the inside of the silicon tube (stress was set to 0 Pa).

Multiple comparison tests were performed for each index ($P < 0.05$, Holm method, one side unpaired t test, F test was preliminarily performed, t verification of Student was adopted in the case of equal variance, and t verification of Welch was adopted in the case of unequal variance). The p values of Samples 1 to 4 were 0.0127, 0.0116, 0.00944, and 0.00782 in order. It was suggested that Samples 1 to 4 had significantly lower stress than the conventional tablets and improved swallowing ability.

The tablets having the same shape as in Example 1 had significantly lower stress than the conventional tablets.

Further, in tablets having the same shape as in Example 1, it was suggested that as the value of the length L/width W of the tablet increases, the stress decreases and the swallowing improves.

From the results of Test Example 5, it was suggested that in tablets having the same shape as in Example 1 as the tablet for oral administration, if the value of the length L/width W of the tablet was at least 1.79 or more, stress was significantly smaller than the conventional tablets and it is easy to swallow the tablet.

On the other hand, in tablets having the same shape as in Example 1, it was suggested that, when the value of the length L/width W of the tablet is less than 1.79, there is a risk that the stress becomes large and it becomes difficult to swallow the tablet than the conventional tablet.

As a reason, it is considered that, when the value of the tablet length L/width W becomes small (when the tablet length L becomes small and the width W becomes large), the tablet becomes easy to rotate in the silicon tube, and a large frictional force is generated.

In particular, since the shape similar to that of Example 1 has a protruding portion, it was considered that, when the tablet rotates, the tablet easily comes into contact with the inside of the silicon tube (it is easy to get caught).

Actually, assuming a human swallowing, in the case of the shape in which the tablet has a protruding portion, as the value of the length L/the width W becomes smaller (as the two axes of the length L and the width W become a close value), it was considered that the tablet is caught in the human pharynx and rotates, turns around in the pharynx, and as a result, in some cases, clogging may occur.

(Summary of Test Examples 1 to 3)

It was found that in order to stably pinch tablets having the same shape as tablets 1 and tablets 101, it is desirable that the height when these tablets are inclined be 3.4 mm or more, and in order to further improve the easiness of pinching, it is desirable that the height when these tablets are inclined be 4.0 mm or more.
Further, it was found that in order to further improve the easiness of pinching, it is preferable that the width W of the tablet be 3.9 mm or more, and it is desirable that the value of the length L/width W be 3.72 or less.

(Summary of Test Examples 4 to 5)

It was suggested that it is desirable to set the value of the length L/width W of the tablet to 1.79 or more in order to improve the swallowing of the tablet having the same shape as the tablet 1 and the tablet 101.
As described above, in order to improve both the easiness of pinching and the swallowing of tablets having the same shape as the tablets 1 and 101, it was suggested that it is desirable that (1) the height when these tablets are inclined be 3.4 mm or more, (2) the width W of the tablet be 3.9 mm or more, and (3) the value of the length L/width W of the tablet be 1.79 or more and 3.72 or less.
In other words, it was suggested that it is desirable to satisfy the following formulas 1 to 4.

$$\text{Formula 1: } L1 \times \sin(180° - \theta_1) \geq 3.4$$

$$\text{Formula 2: } L2 \times \sin(180° - \theta_2) \geq 3.4$$

$$\text{Formula 3: } W \geq 3.9$$

$$\text{Formula 4: } 1.79 \leq L/W \leq 3.72$$

INDUSTRIAL APPLICABILITY

The tablet of the present invention can be easily pinched even if an elderly person or a patient who has a limited range of joint motion is a target, and can be used as a tablet that is hard to roll even when slipping out of the hand. Further, the tablet can also be applied as a tablet for oral administration, taking the swallowing into consideration, even if the elderly person or a rheumatoid arthritis patient complicated with Sjogren's syndrome is a target.

REFERENCE SIGNS LIST

1,101: tablet
10, 110: tablet body portion
11, 111: upper surface
11a, 111a: end portion
12, 112: bottom surface
12a, 112a: end portion
13, 14, 113, 114: side surface
20, 120: protruding portion
21, 121: first protruding portion

21a, 121a: upper surface
21b, 121b: leading end portion
21c: first inclined surface

22, 122: second protruding portion

22a, 122a: bottom surface
22b, 122b: leading end portion
22c: second inclined surface

L: length of tablet

L1: length of bottom surface
L2: length of upper surface

W: width of tablet
H: height of tablet
P: protruding length of protruding portion

P1: protruding length of first protruding portion
P2: protruding length of second protruding portion $\theta$: inclined angle between inclined surface and bottom surface
$\theta_1$: first inclined angle
$\theta_2$: second inclined angle

**Claims**

1. A tablet comprising:

   a tablet body portion having a three dimensional shape; and
   a protruding portion continuously protruding from a side surface of the tablet body portion to an outside in an orthogonal direction to a height direction of the tablet body portion,
   wherein the protruding portion has a first protruding portion and a second protruding portion protruding in opposite directions from each other via the tablet body portion,
   the first protruding portion and the second protruding portion are disposed at different height positions from each other,
   the first protruding portion protrudes from an upper portion of the side surface of the tablet body portion,
   the second protruding portion protrudes from a lower portion of the side surface of the tablet body portion,
   the first protruding portion and the second protruding portion are formed to have a narrower width as the first and the second protruding portions are away from the tablet body portion,
   the first protruding portion is formed such that an upper surface of the first protruding portion is substantially flush with the upper surface of the tablet body portion,
   the second protruding portion is formed such that a bottom surface of the second protruding portion is substantially flush with a bottom surface of the tablet body portion,
   the bottom surface of the first protruding portion extends in a planar form in a positional relation orthogonal or intersecting to the side surface of the tablet body portion, and
   the upper surface of the second protruding portion extends in a planar form in a positional relation orthogonal or intersecting to the side surface of the tablet body portion.

2. The tablet according to claim 1, wherein the tablet is for oral administration,

   the first protruding portion has a first inclined surface which connects an end portion of the bottom surface of the tablet body portion on the first protruding portion side and a leading end portion of the first protruding portion, and which extends in a direction intersecting the bottom surface of the tablet body portion, and
   the second protruding portion has a second inclined surface which connects the end portion of the upper surface of the tablet body portion on the second protruding portion side and a leading end portion of the second protruding portion, and which extends in a direction intersecting the upper surface of the tablet body portion.

3. The tablet according to claim 1 or 2, wherein when a length of the bottom surface of the tablet in the protruding direction of the protruding portion is set as L1 (mm), and a length of the upper surface of the tablet in the protruding direction is set as L2 (mm), and

when an angle formed between an imaginary surface connecting an end portion of the bottom surface on the first protruding portion side and a leading end portion of the first protruding portion, and the bottom surface is set as $\theta_1$ (°), and an angle formed between an imaginary surface connecting an end portion of the upper surface on the second protruding portion side and a leading end portion of the second protruding portion, and the upper surface is set as $\theta_2$ (°),
at least one of the following formulas 1 and 2 are satisfied:

$$\text{Formula 1: } L1 \times \sin(180° - \theta_1) \geq 3.4$$

$$\text{Formula 2: } L2 \times \sin(180° - \theta_2) \geq 3.4$$

4. The tablet according to claim 3, wherein a value obtained by dividing the length L of the tablet by the width W of the tablet is 1.79 or more and 3.72 or less.


**Patentansprüche**

1. Eine Tablette umfassend:

   ein Tablettenkörperteil mit einer dreidimensionalen Form; und
   einen vorstehenden Abschnitt, der kontinuierlich von einer Seitenfläche des Tablettenkörperabschnitts nach außen in einer orthogonalen Richtung zu einer Höhenrichtung des Tablettenkörperabschnitts vorsteht,
   wobei der vorstehende Abschnitt einen ersten vorstehenden Abschnitt und einen zweiten vorstehenden Abschnitt aufweist, die über den Tablettenkörperabschnitt in entgegengesetzten Richtungen voneinander abstehen,
   der erste vorspringende Abschnitt und der zweite vorspringende Abschnitt in unterschiedlichen Höhenpositionen zueinander angeordnet sind ,
   der erste vorspringende Teil von einem oberen Teil der Seitenfläche des Tablettenkörperteils vorsteht,
   der zweite vorspringende Teil von einem unteren Teil der Seitenfläche des Tablettenkörperteils vorsteht,
   der erste vorspringende Abschnitt und der zweite vorspringende Abschnitt so geformt sind, dass sie eine schmalere Breite aufweisen, wenn der erste und der zweite vorspringende Abschnitt vom Tablettenkörperabschnitt entfernt sind,
   der erste vorspringende Abschnitt so geformt ist, dass eine obere Fläche des ersten vorspringenden Abschnitts im Wesentlichen mit der oberen Fläche des Tablettenkörperabschnitts bündig ist,
   der zweite vorspringende Abschnitt so geformt ist, dass eine Bodenfläche des zweiten vorspringenden Abschnitts im Wesentlichen bündig mit einer Bodenfläche des Tablettenkörperabschnitts ist,
   die Bodenfläche des ersten vorstehenden Abschnitts sich in einer ebenen Form in einer Lagebeziehung orthogonal oder schneidend zu der Seitenfläche des Tablettenkörperabschnitts erstreckt, und
   die obere Fläche des zweiten vorstehenden Abschnitts sich in einer ebenen Form in einer Lagebeziehung orthogonal oder schneidend zu der Seitenfläche des Tablettenkörperabschnitts erstreckt.

2. Die Tablette nach Anspruch 1, wobei die Tablette zur oralen Verabreichung bestimmt ist,

   der erste vorstehende Abschnitt eine erste geneigte Fläche aufweist, die einen Endabschnitt der Bodenfläche des Tablettenkörperabschnitts auf der Seite des ersten vorstehenden Abschnitts und einen vorderen Endabschnitt des ersten vorstehenden Abschnitts verbindet und die sich in einer Richtung erstreckt, die die Bodenfläche des Tablettenkörperabschnitts schneidet, und
   der zweite vorstehende Abschnitt eine zweite geneigte Fläche aufweist, die den Endabschnitt der oberen Fläche des Tablettenkörperabschnitts auf der Seite des zweiten vorstehenden Abschnitts und einen vorderen Endabschnitt des zweiten vorstehenden Abschnitts verbindet und die sich in einer Richtung erstreckt, die die obere Fläche des Tablettenkörperabschnitts schneidet.

3. Tablette nach Anspruch 1 oder 2, wobei, wenn eine Länge der unteren Oberfläche der Tablette in der vorstehenden Richtung des vorstehenden Abschnitts als L1 (mm) festgelegt ist und eine Länge der oberen Oberfläche der Tablette in der vorstehenden Richtung als L2 (mm) festgelegt ist, und

wenn ein Winkel, der zwischen einer imaginären Fläche, die einen Endabschnitt der unteren Fläche auf der Seite des ersten vorstehenden Abschnitts, und einen vorderen Endabschnitt des ersten vorstehenden Abschnitts verbindet, und der unteren Fläche gebildet wird, als $\theta_1$ (°) eingestellt ist, und ein Winkel, der zwischen einer imaginären Fläche, die einen Endabschnitt der oberen Fläche auf der Seite des zweiten vorstehenden Abschnitts und einen vorderen Endabschnitt des zweiten vorstehenden Abschnitts verbindet, und der oberen Fläche gebildet wird, als $\theta_2$ (°) eingestellt ist,
mindestens eine der folgenden Formeln 1 und 2 erfüllt ist:

$$\text{Formel 1: } L1 \times \sin(180° - \theta_1) \geq 3.4$$

$$\text{Formel 2: } L2 \times \sin(180° - \theta_2) \geq 3.4$$

**4.** Tablette nach Anspruch 3, wobei ein Wert, der durch Teilen der Länge L der Tablette durch die Breite W der Tablette erhalten wird, 1,79 oder mehr und 3,72 oder weniger beträgt.

**Revendications**

**1.** Un comprimé comprenant :

une partie du corps du comprimé ayant une forme tridimensionnelle; et
une partie en saillie faisant continuellement saillie depuis une surface latérale de la partie de corps du comprimé vers l'extérieur dans une direction orthogonale à une direction de hauteur de la partie du corps de comprimé, dans lequel la partie en saillie a une première partie en saillie et une seconde partie en saillie faisant saillie dans des directions opposées l'une de l'autre via la partie du corps de comprimé,
la première partie saillante et la seconde partie saillante sont disposées à des positions de hauteur différentes l'une de l'autre,
la première partie en saillie fait saillie depuis une partie supérieure de la surface latérale de la partie du corps de comprimé,
la seconde partie en saillie fait saillie depuis une partie inférieure de la surface latérale de la partie du corps de comprimé,
la première partie en saillie et la seconde partie en saillie sont formées pour avoir une largeur plus étroite lorsque les première et seconde parties en saillie sont éloignées de la partie du corps de comprimé,
la première partie en saillie est formée de telle sorte qu'une surface supérieure de la première partie en saillie est sensiblement de niveau avec la surface supérieure de la partie du corps de comprimé,
la seconde partie en saillie est formée de telle sorte qu'une surface inférieure de la seconde partie en saillie est sensiblement de niveau avec une surface inférieure de la partie du corps de comprimé,
la surface inférieure de la première partie en saillie s'étend sous une forme plane dans une relation de position orthogonale ou d'intersection avec la surface latérale de la partie du corps de comprimé, et
la surface supérieure de la seconde partie saillante s'étend sous une forme plane dans une relation de position orthogonale ou d'intersection avec la surface latérale de la partie du corps de comprimé.

**2.** Comprimé selon la revendication 1, dans lequel le comprimé est destiné à une administration orale,

la première partie saillante a une première surface inclinée qui relie une partie d'extrémité de la surface inférieure de la partie du corps de comprimé sur le côté de la première partie saillante et une partie d'extrémité avant de la première partie saillante, et qui s'étend dans une direction coupant la surface inférieure de la partie du corps de comprimé, et
la seconde partie saillante a une seconde surface inclinée qui relie la partie d'extrémité de la surface supérieure de la partie du corps de comprimé sur le côté de la seconde partie saillante et une partie d'extrémité avant de la seconde partie saillante, et qui s'étend dans une direction coupant la surface supérieure de la partie du corps de comprimé.

**3.** Comprimé selon la revendication 1 ou 2, dans lequel lorsqu'une longueur de la surface inférieure du comprimé dans la direction de saillie de la partie en saillie est définie comme L1 (mm), et une longueur de la surface supérieure du comprimé dans la direction de saillie est définie comme L2 (mm), et

lorsqu'un angle formé entre une surface imaginaire reliant une partie d'extrémité de la surface inférieure sur le côté de la première partie saillante et une partie d'extrémité avant de la première partie saillante, et la surface inférieure est défini comme $\theta_1$ (°), et un angle formé entre une surface imaginaire reliant une partie d'extrémité de la surface supérieure sur le côté de la seconde partie saillante et une partie d'extrémité avant de la seconde partie saillante, et la surface supérieure est défini comme $\theta_2$ (°),
au moins une des formules 1 et 2 suivantes est satisfaite :

$$\text{Formule 1: } L1 \times \sin(180°\text{-}\theta_1) \geq 3,4$$

$$\text{Formule 2: } L2 \times \sin(180°\text{-}\theta_2) \geq 3.4$$

4. Comprimé selon la revendication 3, dans lequel une valeur obtenue en divisant la longueur L du comprimé par la largeur W du comprimé est égale ou supérieure à 1,79 et inférieure ou égale à 3,72.

# FIG. 1

# FIG. 2A

# FIG. 2B

# FIG. 3

## FIG. 4A

## FIG. 4B

# FIG. 4C

# FIG. 4D

# FIG. 4E

# FIG. 4F

# FIG. 5

# FIG. 6

# FIG. 7A

# FIG. 7B

# FIG. 7C

# FIG. 7D

# FIG. 7E

# FIG. 7F

FIG. 8A

1

$L1 \times Sin(180° - \theta_1)$

$180° - \theta_1$

FIG. 8B

101

$L1 \times Sin(180° - \theta_1)$

$180° - \theta_1$

121b    112a

# FIG. 9

# FIG. 10

# FIG. 11

# FIG. 12

WORKING TIME OF ORDINARY TABLET = 61.4 [SECOND]

LENGTH L / WIDTH W OF EXAMPLE 1

# FIG. 13

# FIG. 14

# FIG. 15

STRESS OF ORDINARY TABLET = $3.03 \times 10^5$ [Pa]

LENGTH L / WIDTH W OF EXAMPLE 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2386416 A
- JP 3158894 U
- JP 2785049 B
- JP 2013046453 A

**Non-patent literature cited in the description**

- **OWAKI NAOKO.** A Multi-center Study on Convenience of Removing Tablets and Capsules from Heat-sealed Packages. *medical medicine,* 2004, vol. 30 (5), 312-320
- **HOLM S.** A simple sequentially rejective multipletest procedure. *Scand J Stat.,* 1979, vol. 6, 65-70